Europäisches Patentamt

European Patent Office

Office européen des brevets

(11) Publication number: **0 171 077 B1**

(12) **EUROPEAN PATENT SPECIFICATION**

(45) Date of publication of patent specification: **14.11.90**

(21) Application number: **85109936.6**

(22) Date of filing: **07.08.85**

(60) Divisional application **89122016.2 filed on 07/08/85.**

(51) Int. Cl.⁵: **A 61 M 25/01**

(54) Catheter introducer device.

(30) Priority: **07.08.84 JP 165452/84**

(43) Date of publication of application:
**12.02.86 Bulletin 86/07**

(45) Publication of the grant of the patent:
**14.11.90 Bulletin 90/46**

(84) Designated Contracting States:
**BE DE FR GB IT SE**

(56) References cited:
**EP-A-0 064 212**
**WO-A-81/02098**
**DE-A-2 305 640**
**FR-A-2 474 317**
**US-A-3 399 674**
**US-A-3 856 009**

(73) Proprietor: **TERUMO KABUSHIKI KAISHA trading as TERUMO CORPORATION**
**44-1, 2-chome, Hatagaya Shibuya-Ku**
**Tokyo 151 (JP)**

(72) Inventor: **Suzuki, Tatsuo**
**1-5-27-302, Sakuradai Oyamada**
**Machida-shi Tokyo (JP)**
Inventor: **Matsumoto, Atsushi**
**Tajima apt. 202 2-6, 5-chome, Fujimidai**
**Fuji-shi Shizuoka-ken (JP)**

(74) Representative: **Henkel, Feiler, Hänzel & Partner**
**Möhlstrasse 37**
**D-8000 München 80 (DE)**

## Description

The present invention relates to a catheter introducer device defined by the precharacterizing features of claim 1.

A catheter introducer device is known as a device for percutaneous insertion of a catheter into a blood vessel. A catheter introducer device is composed of a sheath portion having a sheath and a sheath hub, and a dilator portion having a tube and a dilator hub. When such a catheter introducer device is used, the dilator portion is set in the sheath portion and the dilator and sheath portions are inserted into a blood vessel via a guide wire.

The dilator and sheath portions can be easily inserted when they are simultaneously rotated during insertion. In a conventional catheter introducer device, no means for simultaneously rotating the sheath and dilator portions is included. In such a conventional catheter introducer, in order to insert the assembly of sheath and dilator portions into a blood vessel while preventing rotation between the sheath and dilator portions, the hubs in the two portions must either be clamped together, or the tube of the dilator portion must be firmly clamped at a postion above the sheath of the sheath portion so as to simultaneously rotate the two portions, thus resulting in a complex procedure.

Yet, even if such complex operation is performed, only the sheath portion can be inserted, the dilator portion being impeded by resistance of tissue or the like.

A catheter introducer device of the kind defined at the preamble is known from the EP—A—64 212 The second hub of this known device is screwed on the first hollow hub. The threads of this screw, which are arranged between the first and second hubs, form first and second means for preventing the relative axial movement and the relative circumferential rotation, if the second hub is completely screwed on the first hub. If, however, the second hub is not completely screwed into the first hub, axial movement as well as circumferential rotation therebetween and hence between the sheath portion and the dilator is not prevented. Further, the second hub needs to be tightly screwed into the first hub in order to prevent loosening of the screw connection. A loose fit of the two hubs, as aforementioned, however, is not suited to prevent axial movement and circumferential rotation.

The catheter introducer device known from the DE—A—2 305 640 comprises a male-female tapering which is intended to hold the two parts of this catheter introducer device together. This tapering constitutes means for preventing relative axial movement and circumferential rotation when tightly assembled. This male-female tapering has a circular cross section and hence does not provide a safe connection for the two parts of the catheter introducer device, which may be loosened upon manipulation of the device.

It is the object of the present invention to provide a catheter introducer device of the kind defined at the preamble with safe means for preventing relative axial movement and circumferential rotation of the sheath portion relative to the dilator.

This object is attained by characterizing features of the claim 1. Advantageous developments of the invention are given by the subclaims.

In accordance with the invention a clamp connection is provided by means of two ribs adjacently arranged in axial direction for a safe coupling of the sheet portion and the dilator in axial direction, thereby safely preventing relative axial movement of these parts. Further in accordance with the invention relative circumferential rotation of said parts is safely prevented by means of non-circular fitting portions provided thereon. Coaction of the non-linear fitting portions with the clamp connection safely prevents loosening of the once assembled parts of the catheter introducer device during manipulation therewith.

This invention can be more fully understood from the following detailed description when taken in conjunction with the accompanying drawings, in which:

Fig. 1 is a side view of an assembled catheter introducer device of the present invention;

Fig. 2 is a partial sectional view of the main body of the device shown in Fig. 1

Figs. 3, 4 and 5 are diagrams for explaining the operation for inserting the device shown in Fig. 1, 2 and 6 into a blood vessel; and

Figs. 6 and 8 are perspective views showing another embodiment of a medical device of the present invention.

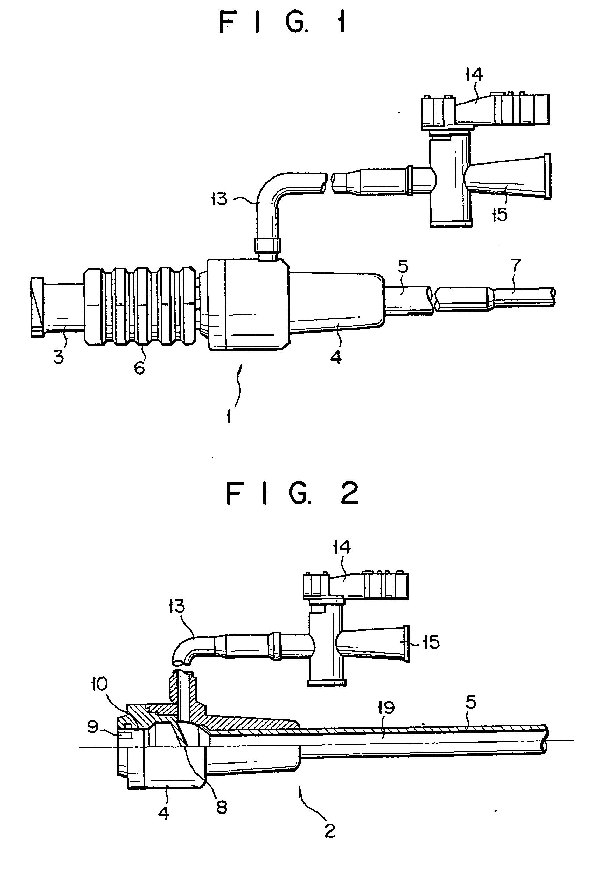

The catheter introducer 1 (Fig. 1) of the present invention incldues a main body 2 (Fig. 2) and a rod member 3. The main body 2 corresponds to the sheath portion, and the rod member 3 corresponds to the dilator portion of a catheter introducer.

The main body 2 has a hub 4 and a sheath 5 having its proximal portion fixed and supported by the hub 4, as shown in Fig. 2. The hub 4 and the sheath 5 have a through path 19 for receiving a tube 7 of the rod member 3.

A valve 8 is mounted, preferably, in the portion of the through path 19 in the hub 4. The valve 8 serves to prevent reverse flow of blood when the medical device main body 2 is inserted into a blood vessel.

The rod member or dilator portion 3 has a hub 6 and a tube 7 fixed and supported thereby. The dilator portion 3 is inserted in and assembled with the main body 2 shown in Fig. 2. With this arrangement, when the main body 2 and the rod member 3 are percutaneously inserted into a blood vessel, they rotate relative to each other and, as such, may not lend themselves to easy insertion, as described above.

In view of this problem, according to the present invention a mechanism for preventing relative rotation between the main body 2 and the dilator portion 3 is incorporated.

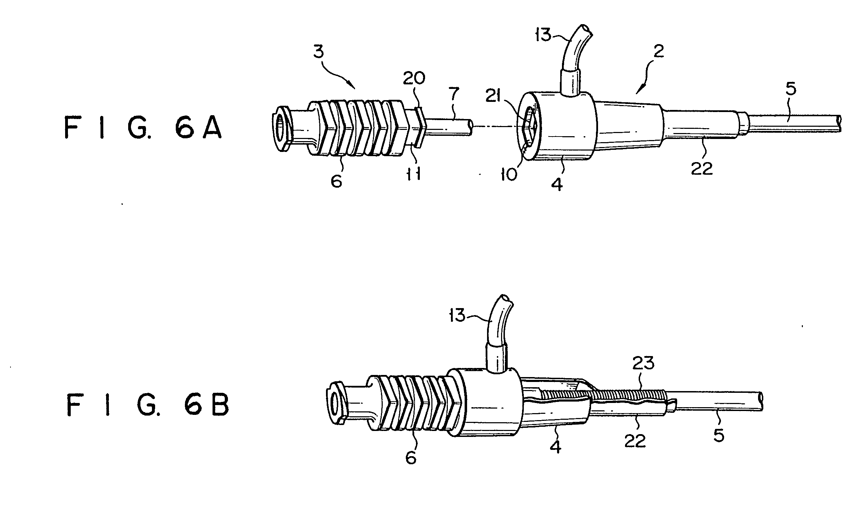

Figs. 6A and 6B show such an arrangement of a mechanism for preventing axial displacement and circumferential rotation.

In the arrangement shown in Fig. 6A, fitting portions 11 and 10 of a rod member 3 and a medical device main body 2 have hexagonal sections to prevent relative rotations therebetween. Ribs 20 and 21 on the fitting portions 11 and 10 engage with each other so as to prevent axial displacement therebetween. The ribs 20 and 21 need be formed to engage with at least parts of the fitting portions. The rib 20 slips over and positions beyond the rib 21, thus preventing the axial movement. It is preferred to form three ribs, instead of the annular rib 20, on the periphery of the portion 11, spaced apart from each other at angular intervals of 120° when the portion 11 is of hexagonal shape. This configuration of the ribs facilitates the slipping-over and pulling-out operations. (Fig. 6B shows the state wherein the fitting portions 11 and 10 are fit together). In the arrangement shown in Fig. 6B, reference numeral 22 denotes a shrinkage tube, and 23 a bending spring.

As described above, the sections of fitting portions of the rod member 3 and the medical device main body 2 are non-circular but can be various shapes, including polygons such as hexagons or ellipses.

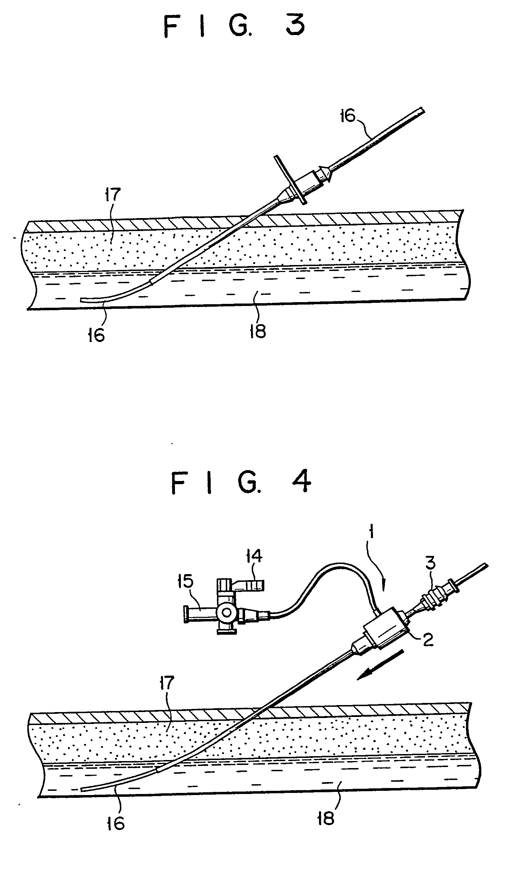

The method of using the medical device of the present invention will be described with reference to Figs. 3 to 5.

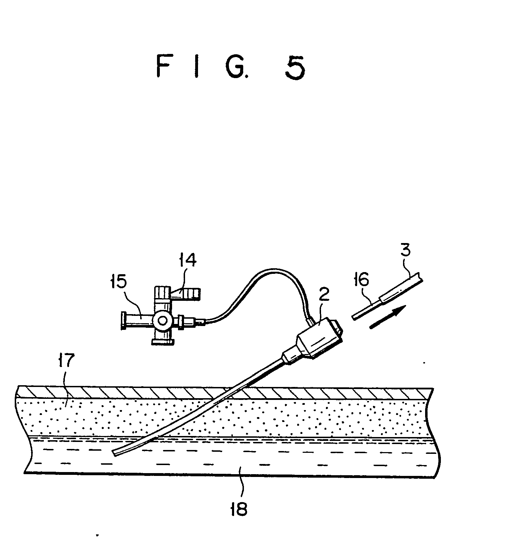

A guide wire 16 is inserted into a blood vessel 18 through a subcutaneous tissue 17 by a suitable method such as the Seldinger method (Fig. 5). Then, the medical device main body 1, obtained by assembling the sheath portion (medical device main body) 2 shown in Fig. 2 and the dilator portion (rod member) 3 shown in Fig. 4 so as to prevent axial movement and circumferential rotation as shown in Fig. 1, is inserted into a blood vessel 18 by inserting the guide wire 16 into the tube 7, the sheath 5 and the through hole in the hubs 4 and 6 (Fig. 6).

In this process, insertion into the blood vessel can be easily performed when the hub 6 of the dilator portion 3 is rotated during insertion. In contrast, insertion of the main body 1 cannot be performed easily with a conventional device, since the conventional device does not include a mechanism for preventing relative rotation between the dilator and sheath portions, as described above.

In the medical device of the present invention, axial movement between the portions 3 and 2 is prevented by a fitting force obtained by the arrangement shown in Fig. 6A and 6B. Therefore, insertion of the medical device main body 1 into the blood vessel 18 through means of the guide wire 16 is facilitated.

Subsequently, the sheath portion 6 is left in the blood vessel 18, and the guide wire 16 and the dilator portion 3 are pulled out (Fig. 5).

The catheter can be easily inserted into the blood vessel 18 through the indwelling sheath portion 2. Since the sheath portion 2 has a check valve 8, reverse flow of blood is prevented. If required, suction of a thrombus or injection of a physiological saline solution can be performed through the lure taper port 15 of the sheath portion 2 before or after insertion of the catheter.

When the resultant assembly is inserted into a blood vessel, as described with reference to Figs. 3 to 5, a suitable portion of the assembly, such as the hub of the rod member, is clamped such that the assembly is inserted while applying a rotational force around the guide wire. The insertion operation which, conventionally, has been complex is, through application of the invention, made easy.

**Claims**

1. A catheter introducer device, comprising:

a sheath portion (2) including a sheath (5) and a first hollow hub (4) fitted to a proximal end of said sheath (5) which is adapted to be inserted into blood vessels (18);

a dilator (3) including a tube (7) detachably insertable into said sheath (5), and a second hub (6) fixed to a proximal end of said tube (7) which is adapted to guide a guided wire (16) therethrough;

first means for preventing relative axial movement of said sheath portion (2) and said dilator (3) when said tube (7) is inserted into said sheath (5) and

second means for preventing relative circumferential rotation of said sheath portion (2) and said dilator (3) when said tube (7) is inserted into said sheath (5),

said first and second means being arranged between said first and second hubs (4, 6), characterized in that said first means includes at least one rib (20) formed on the outer surface of said second hub (6), and at least one rib (21) formed on the inner surface of said first hub (4), said rib (20) formed on the outer surface of said second hub (6) being located to a position beyond said rib (21) formed on the inner surface of said first hub (4) to prevent said axial movement when said tube (7) is inserted into said sheath (5), and

in that said second means includes a female non-circular fitting portion (10) defining the inner surface of said first hub (4), and a male non-circular fitting portion (11) defining the outer surface of said second hub (6), said male fitting portion (11) being fitted into said female fitting portion (10) to prevent said rotation when said tube (7) is inserted into said sheath (5).

2. The catheter according to claim 1, wherein the rib (20) formed on the outer surface of said second hub (6) and the rib (21) formed on the inner surface of said first hub (4) have an annular shape.

3. The catheter according to claim 1 or 2, wherein the sections of said male and female fitting portions (10, 11) are polygons.

4. The catheter according to claim 1 or 2, wherein the sections of said male and female fitting portions (10, 11) are ellipses.

**Patentansprüche**

1. Vorrichtung zum Einführen eines Katheters mit

einem Hülsenabschnitt (2), der eine Hülse (5) einschließt sowie eine erste hohle Nabe (4), die an ein proximales Ende der Hülse (4) angeschlossen ist, das zur Einführung in Blutgefäße ausgelegt ist,

einem Dilator (3), der ein Rohr (7) einschließt, das in die Hülse (5) herausnehmbar einsetzbar ist, sowie eine zweite Nabe (6), die an einem proximalen Ende des Rohrs (7) befestigt ist, das zur Führung eines Führungsdrahtes (16) durch dasselbe ausgelegt ist,

einem ersten Mittel zur Verhinderung einer relativen axialen Bewegung des Hülsenabschnitts (2) und des Dilators (3), wenn das Rohr (7) in die Hülse (5) eingesetzt ist, und

einem zweiten Mittel zur Verhindern einer relativen Umfangsdrehung des Hülsenabschnitts (2) und des Dilators (3), wenn das Rohr (7) in die Hülse (5) eingesetzt ist,

wobei die ersten und zweiten Mittel zwischen den ersten und zweiten Naben (4, 5) angeordnet sind, dadurch gekennzeichnet, daß das erste Mittel wenigstens eine Rippe (20) einschließt, die auf der Außenfläche der zweiten Nabe (6) ausgebildet ist, sowie wenigstens eine Rippe (21), die auf der Innenfläche der ersten Nabe (4) ausgebildet ist, wobei die auf der Außenfläche der zweiten Nabe (6) ausgebildete Rippe (20) an einer Position jenseits der auf der Innenfläche der ersten Nabe (4) ausgebildeten Rippe (21) angeordnet ist, um besagte axiale Bewegung zu verhindern, wenn das Rohr (7) in die Hülse (5) eingesetzt ist, und daß das zweite Mittel einen weiblichen nichtkreisförmigen Paßabschnitt (10) einschließt, der die Innenfläche der ersten Nabe (4) festlegt, sowie einen männlichen nichtkreisförmigen Paßabschnitt (11), der die Außenfläche der zweiten Nabe (6) festlegt, wobei der männliche Paßabschnitt (11) in den weiblichen Paßabschnitt (10) eingepaßt ist, um besagte Drehung zu verhindern, wenn das Rohr (7) in die Hülse eingesetzt ist.

2. Katheter nach Anspruch 1, bei dem die auf der Außenfläche der zweiten Nabe (6) ausgebildete Rippe (20) und die auf der Innenfläche der ersten Nabe (4) ausgebildete Rippe (21) eine ringförmige Gestalt aufweisen.

3. Katheter nach Anspruch 1 oder 2, bei dem die Querschnitte der weiblichen und männlichen Paßabschnitte (10, 11) Polygone sind.

4. Katheter nach Anspruch 1 oder 2, bei dem die Querschnitte der weiblichen und männlichen Paßabschnitte (10, 11) Ellipsen sind.

**Revendications**

1. Dispositif introducteur de cathéter, comprenant:

une partie gaine (2) comprenant une gaine (5) et un premier embout creux (4) enmanché dans l'extrémité proximale de ladite gaine (5) qui est adaptée pour être insérée dans des vaisseaux sanguins (18);

un élément de dilatation (3) comprenant un tube (7) insérable de façon séparable dans ladite gaine (5), et un second embout (6) fixé à l'extrémité proximale dudit tube (7) qui est adapté pour guider un fil (16) de guidage qui le traverse;

un premier moyen pour empêcher un déplacement axial relatif de la partie gaine (2) et de l'élément de dilatation (3) lorsque le tube (7) est inséré dans la gaine (5); et

un second moyen pour empêcher une rotation circonférentielle relative de la partie gaine (2) et de l'élément de dilatation (3) lorsque le tube (7) est inséré dans la gaine (5);

lesdits premier et second moyens étant disposés entre des premier et second embouts (4, 6), caractérisé en ce que le premier moyen comprend au moins une nervure (20) formé sur la surface extérieure dudit second embout (6) et au moins une nervure (21) formée sur la surface intérieure du premier embout (4), ladite nervure (20) formée sur la surface extérieure du second embout (6) étant placée à un endroit situé au-delà de la nervure (21) formée sur la surface intérieure du premier moyeu (4) pour empêcher ledit déplacement axial lorsque le tube (7) est inséré dans la gaine (5), et

en ce que le second moyen comprend une partie d'accouplement femelle non circulaire (10) définissant la surface intérieure du premier embout (4) et une partie d'accouplement mâle non circulaire (11) définissant la surface extérieure du second embout (6), ladite partie d'accouplement mâle (11) étant enmanchée dans la partie d'accouplement femelle (10) pour empêcher ladite rotation lorsque le tube (7) est inséré dans la gaine (5).

2. Cathéter selon la revendication 1, dans lequel la nervure (20) formée sur la surface extérieure du second embout (6) et la nervure (21) formée sur la surface intérieure du premier embout (4) ont une forme annulaire.

3. Cathéter selon la revendication 1 ou 2, dans lequel les sections desdites parties d'accouplement mâle et femelle (10, 11) sont polygonales.

4. Cathéter selon la revendication 1 ou 2, dans lequel la section desdites parties d'accouplement mâle et femelle (10, 11) sont elliptiques.

# F I G. 1

# F I G. 2

1

# F I G. 3

# F I G. 4

# F I G. 5

F I G. 6A

3

20

7

21

13

2

5

6

11

10

4

22

F I G. 6B

13

23

6

4

22

5

EP 0 171 077 B1